# EUROPEAN PATENT SPECIFICATION

(11) **EP 0 699 232 B1**
(45) Date of publication and mention of the grant of the patent: **28.07.1999**
(21) Application number: 94917979.0
(22) Date of filing: 12.05.1994
(51) Int. Cl.: C11D 3/39

(54) **BLEACHING COMPOSITIONS COMPRISING N-ACYL CAPROLACTAM ACTIVATORS**
BLEICHMITTELZUSAMMENSETZUNGEN ENTHALTEND N-ACYLCAPROLACTAM AKTIVATOREN
COMPOSITIONS DE BLANCHIMENT COMPRENANT DES ADJUVANTS A BASE DE N-ACYL CAPROLACTAME

(30) Priority: 20.05.1993 US 64624
(43) Date of publication of application: 06.03.1996
(73) Proprietor: THE PROCTER & GAMBLE COMPANY, Cincinnati, Ohio 45202 (US)
(72) Inventor: WILLEY, Alan, David, Cincinnati, OH 45202 (US); BURNS, Michael, Eugene, Hamilton, OH 45011 (US); COLLINS, Jerome, Howard, Cincinnati, OH 45224 (US)
(74) Representative: Peet, Jillian Wendy
(86) International application number: US9405369
(87) International publication number: WO9428103

(56) References cited:
- EP-A- 0 122 763
- FR-A- 2 388 924

## Description

### FIELD OF THE INVENTION

The present invention relates to laundry detergents with activated bleaching systems.

### BACKGROUND OF THE INVENTION

It has long been known that peroxygen bleaches are effective for stain and/or soil removal from fabrics, but that such bleaches are temperature dependent. At a laundry liquor temperature of 60°C, peroxygen bleaches are only partially effective. As the laundry liquor temperature is lowered below 60°C, peroxygen bleaches become relatively ineffective. As a consequence, there has been a substantial amount of industrial research to develop bleaching systems which contain an activator that renders peroxygen bleaches effective at laundry liquor temperatures below 60°C.

Numerous substances have been disclosed in the art as effective bleach activators. One widely-used bleach activator is tetraacetyl ethylene diamine (TAED). TAED provides effective hydrophilic cleaning especially on beverage stains, but has limited performance on dingy, yellow stains such as those resulting from body oils. Another type of activator, such as non-anoyloxybenzenesulfonate (NOBS) and other activators which generally comprise long chain alkyl moieties, is hydrophobic in nature and provides excellent performance on dingy stains. However, many of the hydrophobic activators developed thus far can promote damage to natural rubber parts used in certain washing machines and to natural rubber articles exposed to the activators. Because of these negative effects on natural rubber washing machine parts and articles, the selection of such detergent-added bleaching systems has been limited. This is especially true for European detergents/bleaches, since many washing machines manufactured in Europe have been equipped with key parts, such as sump hoses and motor gaskets, made of natural rubber.

A need, therefore, exists for a bleaching system which provides efficient stain removal without substantially damaging natural rubber articles or the natural rubber parts found in washing machines.

It has now been determined that in conventional bleaching systems, particularly those comprising a hydrophobic bleach activator and a source of hydrogen peroxide, the bleach activator undergoes perhydrolysis to form a peroxyacid bleaching agent. A by-product of the perhydrolysis reaction between such bleach activators and hydrogen peroxide is a diacylperoxide (DAP) species. It has now further been discovered that the DAP's derived from hydrophobic activators tend to be insoluble, poorly dispersible, oily materials which form a residue which can deposit on the natural rubber machine parts that are exposed to the laundry liquor. The oily DAP residue can form a film on the natural rubber parts and promote free radical and peroxide damage to the rubber, which eventually leads to failure of the part. This is particularly true of rubber parts which have prolonged exposure to the laundry liquor, such as sump hoses.

By the present invention, is has now been discovered that specific bleach activators derived from N-acyl caprolactams form peroxyacids upon perhydrolysis without the production of oily, harmful DAP's. Without intending to be limited by theory, it is believed that the specific bleach activators employed herein provide good cleaning performance with safety to natural rubber, since they do not expose the natural rubber machine parts to DAP oxidation. Whatever the reason, the rubber machine parts remain substantially undamaged by the bleaching systems of the present invention.

The present invention thus solves the long-standing need for an effective hydrophobic bleaching system which does not promote free radical and peroxide damage to natural rubber parts in washing machines or damage to natural rubber articles. The invention provides a method of cleaning fabrics with a bleaching system in washing machines which have parts made of natural rubber, such that the natural rubber is substantially undamaged by the bleaching system.

### BACKGROUND ART

U.S. Patent 4,545,784, Sanderson, issued October 8, 1985, and equivalent to EPA, 122, 763, discloses the adsorption of activators including N-acyl caprolactams onto sodium perborate monohydrate.

### SUMMARY OF THE INVENTION

The present invention relates to specific hydrophobic caprolactam bleach activators used in combination with a source of hydrogen peroxide and, with hydrophilic bleach activators. The caprolactam bleach activators are described below. Additionally, the invention relates to benzoyl caprolactam bleach activators which provide both hydrophobic and hydrophilic bleaching activity.

The present invention also relates to a method for cleaning fabrics in automatic washing machines having parts made of natural rubber which is susceptible to oxidative degradation. The method comprises agitating fabrics in said washing machine in an aqueous liquor comprising a detergent composition which comprises conventional detergent ingredients and a bleaching system which comprises a bleach activator as herein above that reacts with a peroxide source in said aqueous liquor to yield a peroxyacid without the formation of harmful diacylperoxide (DAP) such that said natural rubber parts are substantially undamaged. Said bleaching system preferably comprises:
a) at least 0.1%, preferably from 1% to 75%, by weight, of a peroxygen bleaching compound capable of yielding hydrogen peroxide in an aqueous solution; and
b) at least 0.1%, preferably from 0.1% to 50%, by weight, of one or more N-acyl caprolactam bleach activators. Selected from the group consisting of benzoyl caprolactam, 3,5,5-trimethylhexanoyl caprolactam, nonanoyl caprolactam, decanoyl caprolactam and undecenoyl caprolactam.

The acyl moieties of said N-acyl caprolactam bleach activators have the formula R¹-CO- wherein R¹ is phenyl, octyl, nonyl, decenyl and 2,4,4-trimethylpentyl.

The peroxygen bleaching compound is selected from any convenient source of peroxide, including members selected from the group consisting of sodium perborate monohydrate, sodium perborate tetrahydrate, sodium pyrophosphate peroxyhydrate, urea peroxyhydrate, sodium percarbonate, sodium peroxide and mixtures thereof. Preferred peroxygen bleaching compounds are selected from the group consisting of perborate salts and percarbonate salts, including sodium perborate monohydrate, sodium percarbonate, sodium perborate tetrahydrate and mixtures thereof.

The N-acyl caprolactam activators herein can be used in combination with rubber-safe, non-caprolactam, hydrophilic activators such as TAED, typically at weight ratios of caprolactam:TAED in the range of 1:5 to 5:1, preferably about 1:1.

The method of washing fabrics with the bleaching system employed herein simply comprises contacting said fabrics, preferably with agitation, with an aqueous laundry liquor containing at least 300 ppm of conventional detergent ingredients, at least 25 ppm of the bleaching compound and at least 25 ppm of the bleach activator. Preferably, said aqueous liquor contains from 900 ppm to 20,000 ppm of conventional detergent ingredients, from 100 ppm to 25,000 ppm of the bleaching compound and from 100 ppm to 2,500 ppm of the bleach activator. The method can be carried out at temperatures below 60°C but, of course, is quite effective and is still safe to rubber parts at laundry temperatures up to the boil. The conventional detergent ingredients and bleaching system are typically combined in a detergent composition.

The conventional detergent ingredients employed in said method comprise from 1% to 99.8%, preferably from 5% to 80%, by weight of detergent composition, of a detersive surfactant. Optionally, detergent ingredients comprise from 5% to 80%, by weight of detergent composition, of a detergent builder. Other conventional detersive adjuncts are optionally, but preferably, used in the fully-formulated detergent/bleach compositions provided by this invention.

All percentages, ratios, and proportions herein are by weight, unless otherwise specified.

### DETAILED DESCRIPTION OF THE INVENTION

The bleaching system used in the practice of this invention is safe to natural rubber machine parts and other natural rubber articles exposed to the bleaching system, including fabrics containing natural rubber and natural rubber elastic materials. Additionally, the bleaching system provides not only effective and efficient bleaching of conventional stains and soils on fabrics, but also effective bleaching of so-called "dingy" soils. Dingy soils are those that build up on textiles after numerous cycles of usage and washing, and result in a gray or yellow tint on white fabrics. These soils tend to be a blend of particulate and greasy materials. The removal of this type of soil is sometimes referred to as "dingy fabric clean-up".

The bleaching systems and activators herein afford additional advantages in that, unexpectedly, they are safer to fabrics and cause less color damage than other activators when used in the manner provided by this invention.

The bleaching mechanism and, in particular, the surface bleaching mechanism are not completely understood. However, it is generally believed that the bleach activator undergoes nucleophilic attack by a perhydroxide anion, which is generated from the hydrogen peroxide evolved by the peroxygen bleaching compound, to form a peroxycarboxylic acid. This reaction is commonly referred to as perhydrolysis. It is believed that during perhydrolysis, the caprolactam moiety acts to provide a peracid bleaching species without formation of harmful DAP's. It is further believed that the bleach activators within the invention can render peroxygen bleaches more efficient even at laundry liquor temperatures wherein bleach activators are not necessary to activate the bleach, i.e., above about 60°C. Therefore, with bleach systems of the invention, less peroxygen bleach is required to get the same level of surface bleaching performance as is obtained with the peroxygen bleach alone.

The components of the bleaching systems herein comprise the bleach activator and the peroxide source, as described hereinafter.

### Bleach Activators

The bleach activators employed in the present invention are N-acyl caprolactams of the formula: 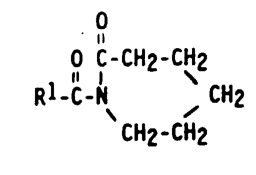 wherein 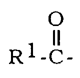 is a benzoylgroup, a 3,5,5 trimethylhexanoyl group, a nonanoyl group, a decanoyl group or an undecenoyl group. Caprolactam activators wherein the R¹ moiety contains at least 6, preferably from 6 to 12, carbon atoms provide hydrophobic bleaching which affords dingy fabric clean-up, as noted above. Caprolactam activators wherein R¹ comprises H or from 1 to 6 carbon atoms provide hydrophilic bleaching species which are particularly efficient for bleaching beverage stains. Mixtures of hydrophobic and hydrophilic caprolactams, typically at weight ratios of 1:5 to 5:1, preferably 1:1, can be used herein for mixed stain removal benefits. Since neither the hydrophobic nor the hydrophilic caprolactams form the undesirable DAP species, both types are safe for use in contact with natural rubber.

Highly preferred hydrophilic N-acyl caprolactams are selected from the group consisting of formyl caprolactam, acetyl caprolactam, and propinoyl caprolactam.

Benzoyl caprolactam, i.e., wherein R¹ is a phenyl substituent, has now been found to be unique among the bleach activator compounds, inasmuch as it appears to exhibit both hydrophobic and hydrophilic bleaching activity. This hydrophobic/hydrophilic bleaching capability makes benzoyl caprolactam the activator of choice for the formulator who is seeking broad spectrum bleaching activity, but wishes to use only a single activator to simplify formulation work.

Some bleaching and cleaning operations are conducted under usage conditions in which the aqueous laundry liquor is at a relatively high temperature, e.g., 80°C to the boil, such as in some European-type washing machines. In such circumstances, there is some prospect that malodors may be perceived by the user. While not intending to be limited by theory, it is believed that such malodors could be caused by volatilization of straight-chain C₆-C₉ fatty acids from the spent activator compound herein. To minimize this problem, bleach activators wherein R¹ is a branchedchain C₆-C₉ moiety can be employed. According to the present invention it is used 3,5,5-trimethylhexanoyl caprolactam.

Methods for making N-acyl caprolactams are well known in the art. Examples I and II, included below, illustrate preferred laboratory syntheses. Contrary to the teachings of U.S. Pat. 4,545,784, cited above, the bleach activator is preferably not absorbed onto the peroxygen bleaching compound. To do so in the presence of other organic detersive ingredients could cause safety problems.

The N-acyl caprolactam bleach activator will usually comprise at least 0.1%, preferably from 0.1% to 50%, more preferably from 1% to 30%, most preferably from 3% to 25%, by weight of bleaching system or detergent composition.

When the activators are used, optimum surface bleaching performance is obtained with washing solutions wherein the pH of such solution is between 8.5 and 10.5, preferably between 9.5 and 10.5, in order to facilitate the perhydrolysis reaction. Such pH can be obtained with substances commonly known as buffering agents, which are optional components of the bleaching systems herein.

### The Peroxygen Bleaching Compound

The peroxygen bleaching compounds useful herein are those capable of yielding hydrogen peroxide in an aqueous liquor. These compounds are well known in the art and include hydrogen peroxide and the alkali metal peroxides, organic peroxide bleaching compounds such as urea peroxide, and inorganic persalt bleaching compounds, such as the alkali metal perborates, percarbonates, perphosphates, and the like. Mixtures of two or more such bleaching compounds can also be used, if desired.

Preferred peroxygen bleaching compounds include sodium perborate, commercially available in the form of mono-, tri-, and tetra-hydrate, sodium pyrophosphate peroxyhydrate, urea peroxyhydrate, sodium percarbonate, and sodium peroxide. Particularly preferred are sodium perborate tetrahydrate, sodium perborate monohydrate and sodium percarbonate. Percarbonate is especially preferred because it is very stable during storage and yet still dissolves very quickly in the bleaching liquor. It is believed that such rapid dissolution results in the formation of higher levels of percarboxylic acid and, thus, enhanced surface bleaching performance.

Highly preferred percarbonate can be in uncoated or coated form. The average particle size of uncoated percarbonate ranges from about 400 to about 1200 microns, most preferably from about 400 to about 600 microns. If coated percarbonate is used, the preferred coating materials include mixtures of carbonate and sulphate, silicate, borosilicate, or fatty carboxylic acids.

The peroxygen bleaching compound will comprise usually at least 0.1%, preferably from 1% to 75%, more preferably from 3% to 40%, most preferably from 3% to 25%, by weight of bleaching system or detergent composition.

The weight ratio of bleach activator to peroxygen bleaching compound in the bleaching system ranges suitably from 2:1 to 1:5. In preferred embodiments, the ratio ranges from 1:1 to 1:3.

The bleach activator/bleaching compound systems herein are useful per se as bleaches. However, such bleaching systems are especially useful in compositions which also comprise various detersive adjuncts such as surfactants, builders and enzymes as disclosed hereinafter.

### Detersive Surfactant

The amount of detersive surfactant included in the fully-formulated detergent compositions afforded by the present invention can vary from 1% to 99.8% depending upon the particular surfactants used and the effects desired. Preferably, the detersive surfactants comprise from 5% to 80% by weight of the detergent ingredients.

The detersive surfactant can be nonionic, anionic, ampholytic, zwitterionic, or cationic. Mixtures of these surfactants can also be used. Preferred detergent compositions comprise anionic detersive surfactants or mixtures of anionic surfactants with other surfactants, especially nonionic surfactants.

Nonlimiting examples of surfactants useful herein include the conventional C₁₁-C₁₈ alkyl benzene sulfonates and primary, secondary, and random alkyl sulfates, the C₁₀-C₁₈ alkyl alkoxy sulfates, the C₁₀-C₁₈ alkyl polyglycosides and their corresponding sulfated polyglycosides, C₁₂-C₁₈ alpha-sulfonated fatty acid esters, C₁₂-C₁₈ alkyl and alkyl phenol alkoxylates (especially ethoxylates and mixed ethoxy/propoxy), C₁₂-C₁₈ betaines and sulfobetaines ("sultaines") and C₁₀-C₁₈ amine oxides. Other conventional useful surfactants are listed in standard texts.

One particular class of adjunct nonionic surfactants especially useful herein comprises the polyhydroxy fatty acid amides of the formula: 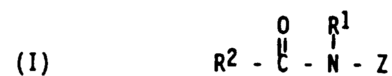 wherein: R¹ is H, C₁-C₈ hydrocarbyl, 2-hydroxyethyl, 2-hydroxypropyl, or a mixture thereof, preferably C₁-C₄ alkyl, more preferably C₁ or C₂ alkyl, most preferably C₁ alkyl (i.e., methyl); and R² is a C₅-C₃₂ hydrocarbyl moiety, preferably straight chain C₇-C₁₉ alkyl or alkenyl, more preferably straight chain C₉-C₁₇ alkyl or alkenyl, most preferably straight chain C₁₁-C₁₉ alkyl or alkenyl, or mixture thereof; and Z is a polyhydroxyhydrocarbyl moiety having a linear hydrocarbyl chain with at least 2 (in the case of glyceraldehyde) or at least 3 hydroxyls (in the case of other reducing sugars) directly connected to the chain, or an alkoxylated derivative (preferably ethoxylated or propoxylated) thereof. Z preferably will be derived from a reducing sugar in a reductive amination reaction; more preferably Z is a glycityl moiety. Suitable reducing sugars include glucose, fructose, maltose, lactose, galactose, mannose, and xylose, as well as glyceraldehyde. As raw materials, high dextrose corn syrup, high fructose corn syrup, and high maltose corn syrup can be utilized as well as the individual sugars listed above. These corn syrups may yield a mix of sugar components for Z. It should be understood that it is by no means intended to exclude other suitable raw materials. Z preferably will be selected from the group consisting of -CH₂-(CHOH)ₙ-CH₂OH, -CH(CH₂OH)-(CHOH)ₙ₋₁-CH₂OH, -CH₂-(CHOH)₂(CHOR')(CHOH)-CH₂OH, where n is an integer from 1 to 5, inclusive, and R' is H or a cyclic mono- or polysaccharide, and alkoxylated derivatives thereof. Most preferred are glycityls wherein n is 4, particularly -CH₂-(CHOH)₄-CH₂OH.

In Formula (I), R¹ can be, for example, N-methyl, N-ethyl, N-propyl, N-isopropyl, N-butyl, N-isobutyl, N-2-hydroxy ethyl, or N-2-hydroxy propyl. For highest sudsing, R¹ is preferably methyl or hydroxyalkyl. If lower sudsing is desired, R¹ is preferably C₂-C₈ alkyl, especially n-propyl, iso-propyl, n-butyl, iso-butyl, pentyl, hexyl and 2-ethyl hexyl.

R²-CO-N< can be, for example, cocamide, stearamide, oleamide, lauramide, myristamide, capricamide, palmitamide or tallowamide.

### Detergent Builders

Optional detergent ingredients employed in the present invention contain inorganic and/or organic detergent builders to assist in mineral hardness control. If used, these builders comprise from 5% to 80% by weight of the detergent compositions.

Inorganic detergent builders include, but are not limited to, the alkali metal, ammonium and alkanolammonium salts of polyphosphates (exemplified by the tripolyphosphates, pyrophosphates, and glassy polymeric meta-phosphates), phosphonates, phytic acid, silicates, carbonates (including bicarbonates and sesquicarbonates), sulphates, and aluminosilicates. However, non-phosphate builders are required in some locales.

Examples of silicate builders are the alkali metal silicates, particularly those having a SiO₂:Na₂O ratio in the range 1.6:1 to 3.2:1 and layered silicates, such as the layered sodium silicates described in U.S. Patent 4,664,839, issued May 12, 1987 to H. P. Rieck, available from Hoechst under the trademark "SKS"; SKS-6 is an especially preferred layered silicate builder.

Carbonate builders, especially a finely ground calcium carbonate with surface area greater than 10 m²/g, are preferred builders that can be used in granular compositions. The density of such alkali metal carbonate built detergents can be in the range of 450-850 g/l with the moisture content preferably below 4%.

Examples of carbonate builders are the alkaline earth and alkali metal carbonates as disclosed in German Patent Application No. 2,321,001 published on November 15, 1973.

Aluminosilicate builders are especially useful in the present invention. Preferred aluminosilicates are zeolite builders which have the formula:

Na_{z}[(AlO₂)_{z} (SiO₂)_{y}]·xH₂O

wherein z and y are integers of at least 6, the molar ratio of z to y is in the range from 1.0 to 0.5, and x is an integer from 15 to 264.

Useful aluminosilicate ion exchange materials are commercially available. These aluminosilicates can be crystalline or amorphous in structure and can be naturally-occurring aluminosilicates or synthetically derived. A method for producing aluminosilicate ion exchange materials is disclosed in U.S. Patent 3,985,669, Krummel, et al, issued October 12, 1976. Preferred synthetic crystalline aluminosilicate ion exchange materials useful herein are available under the designations Zeolite A, Zeolite P (B), (including those disclosed in EPA 384,070), and Zeolite X. Preferably, the aluminosilicate has a particle size of 0.1-10 microns in diameter.

Organic detergent builders suitable for the purposes of the present invention include, but are not restricted to, a wide variety of polycarboxylate compounds, such as ether polycarboxylates, including oxydisuccinate, as disclosed in Berg, U.S. Patent 3,128,287, issued April 7, 1964, and Lamberti et al, U.S. Patent 3,635,830, issued January 18, 1972. See also "TMS/TDS" builders of U.S. Patent 4,663,071, issued to Bush et al, on May 5, 1987.

Other useful detergent builders include the ether hydroxy-polycarboxylates, copolymers of maleic anhydride with ethylene or vinyl methyl ether, 1, 3, 5-trihydroxy benzene-2, 4, 6-trisulphonic acid, and carboxymethyloxysuccinic acid, the various alkali metal, ammonium and substituted ammonium salts of polyacetic acids such as ethylenediamine tetraacetic acid and nitrilotriacetic acid, as well as polycarboxylates such as mellitic acid, succinic acid, oxydisuccinic acid, polymaleic acid, benzene 1,3,5-tricarboxylic acid, carboxymethyloxysuccinic acid, and soluble salts thereof.

Citrate builders, e.g., citric acid and soluble salts thereof (particularly sodium salt), are preferred polycarboxylate builders that can also be used in granular compositions, especially in combination with zeolite and/or layered silicate builders.

Also suitable in the detergent compositions of the present invention are the 3,3-dicarboxy-4-oxa-1,6-hexanedioates and the related compounds disclosed in U.S. Patent 4,566,984, Bush, issued January 28, 1986.

In situations where phosphorus-based builders can be used, and especially in the formulation of bars used for hand-laundering operations, the various alkali metal phosphates such as the well-know sodium tripolyphosphates, sodium pyrophosphate and sodium orthophosphate can be used. Phosphonate builders such as ethane-1-hydroxy-1,1-diphosphonate and other known phosphonates (see, for example, U.S. Patents 3,159,581; 3,213,030; 3,422,021; 3,400,148 and 3,422,137) can also be used.

### Optional Detersive Adjuncts

As a preferred embodiment, the conventional detergent ingredients employed herein can be selected from typical detergent composition components such as detersive surfactants and detergent builders. Optionally, the detergent ingredients can include one or more other detersive adjuncts or other materials for assisting or enhancing cleaning performance, treatment of the substrate to be cleaned, or to modify the aesthetics of the detergent composition. Usual detersive adjuncts of detergent compositions include the ingredients set forth in U.S. Pat. No. 3,936,537, Baskerville et al.

Adjuncts which can also be included in detergent compositions employed in the present invention, in their conventional art-established levels for use (generally from 0% to 20% of the detergent ingredients, preferably from 0.5% to 10%), include enzymes, especially proteases, cellulases and lipases, color speckles, suds boosters, suds suppressors, antitarnish and/or anticorrosion agents, soil-suspending agents, soil release agents, dyes, fillers, optical brighteners, germicides, alkalinity sources, hydrotropes, antioxidants, enzyme stabilizing agents, perfumes, solvents, solubilizing agents, clay soil removal/antiredeposition agents, polymeric dispersing agents, processing aids and fabric softening components static control agents.

Bleach systems optionally, but preferably, will also comprise a chelant which not only enhances bleach stability by scavenging heavy metal ions which tend to decompose bleaches, but also assists in the removal of polyphenolic stains such as tea stains, and the like. Various chelants, including the aminophosphonates, available as DEQUEST from Monsanto, the nitrilotriacetates, the hydroxyethylethylenediamine triacetates, and the like, are known for such use. Preferred biodegradable, non-phosphorus chelants include ethylenediamine disuccinate ("EDDS"; see U.S. Patent 4,704,233, Hartman and Perkins), ethylenediamine-N,N'-diglutamate (EDDG) and 2-hydroxypropylenediamine-N,N'-disuccinate (HPDDS) compounds. Such chelants can be used in their alkali or alkaline earth metal salts, typically at levels from 0.1% to 10% of the present compositions.

Optionally, the detergent compositions employed herein can comprise, in addition to the bleaching system of the present invention, one or more other conventional bleaching agents, activators, or stabilizers which do not react with or otherwise harm natural rubber. In general, the formulator will ensure that the bleach compounds used are compatible with the detergent formulation. Conventional tests, such as tests of bleach activity on storage in the presence of the separate or fully-formulated ingredients, can be used for this purpose.

Specific examples of optional bleaching activators for incorporation in this invention include tetraacetyl ethylene diamine (TAED), the benzoxazin-type bleaching activators disclosed in U.S. Patent 4,966,723, Hodge et al, issued Oct. 30, 1990, and the bleaching agents and activators disclosed in U.S. Patent 4,634,551, Burns et al, issued Jan. 6, 1987.

Such bleaching compounds and agents can be optionally included in detergent compositions in their conventional art-established levels of use, generally from 0% to 15%, by weight of detergent composition.

Bleaching activators of the invention are especially useful in conventional laundry detergent compositions such as those typically found in granular detergents or laundry bars. U.S. Patent 3,178,370, Okenfuss, issued April 13, 1965, describes laundry detergent bars and processes for making them. Philippine Patent 13,778, Anderson, issued Sept. 23, 1980, describes synthetic detergent laundry bars. Methods for making laundry detergent bars by various extrusion methods are well known in the art.

The following examples are given to further illustrate the present invention, but are not intended to be limiting thereof.

### EXAMPLE I

Synthesis of Nonanoyl Caprolactam - To a two liter three necked round bottomed flask equipped with a condenser, overhead stirrer and 250ml addition funnel is charged 56.6g (0.5 moles) caprolactam, 55.7g (0.55 moles) triethylamine and 1 liter of dioxane; the resulting solution is heated to reflux (120°C). A solution of 88.4g (0.5 moles) nonanoyl chloride dissolved in 200ml of dioxane is then added over 30 minutes, and the mixture is refluxed for a further 6 hours. The reaction mixture is then cooled, filtered, and the solvent removed by rotary evaporation to yield 120.5g of the product as a dark oil. This crude product is then dissolved in diethyl ether, washed with 3x50ml aliquots of water, dried over magnesium sulphate, and the solvent removed by rotary evaporation to yield 81.84g (65% theoretical yield) of product which is shown by NMR to be 90% pure, with the remaining material being nonanoic acid.

### EXAMPLE II

Synthesis of Benzoyl Caprolactam - To a two liter three necked round bottomed flask equipped with a condenser, overhead stirrer and 250ml addition funnel is charged 68.2g (0.6 moles) caprolactam, 70g (0.7 moles) triethylamine and 1 liter of dioxane; the resulting solution is heated to reflux (120°C). A solution of 84.4g (0.6 moles) benzoyl chloride dissolved in 200ml of dioxane is then added over 30 minutes, and the mixture is refluxed for a further 6 hours. The reaction mixture is then cooled, filtered, and the solvent removed by rotary evaporation to yield 121.7g of the product as an oil which crystallizes on standing. This crude product is then redissolved in toluene and precipitated with hexane, yielding 103g (79% theoretical yield) of a white solid which which is shown by NMR to be over 95% pure, with the remaining material being benzoic acid.

### EXAMPLE III (Not within the scope of the invention)

A granular detergent composition is prepared comprising the following ingredients.

| Component | Weight % |
|---|---|
| C₁₂ linear alkyl benzene sulfonate | 22 |
| Phosphate (as sodium tripolyphosphate) | 30 |
| Sodium carbonate | 14 |
| Sodium silicate | 3 |
| Nonanoyl caprolactam | 5 |
| Sodium percarbonate | 10 |
| Ethylenediamine disuccinate chelant (EDDS) | 0.4 |
| Sodium sulfate | 5.5 |
| Minors, filler* and water | Balance to 100% |

| | |
|---|---|
| *Can be selected from convenient materials such as CaCO₃, talc, clay, silicates, and the like. | |

In testing the bleaching performance and effect on natural rubber washing machine parts, the following test method is used:

Aqueous crutcher mixes of heat and alkali stable components of the detergent composition are prepared and spray-dried, and the other ingredients are admixed so that they contain the ingredients tabulated at the levels shown.

The detergent granules with bleach activator are added together with 5 lb. (2.3 kg) of previously laundered fabrics, including natural rubber articles such as elastic fabrics, to an automatic washing machine equipped with a natural rubber sump hose. Actual weights of detergent and bleach activator are taken to provide a 950 ppm concentration of the former and 50 ppm concentration of the latter in the 17 gallon (65 1) water-fill machine. The water used has 119.7 mg/l (7 grains/gallon) hardness and a pH of 7 to 7.5 prior to (about 9 to about 10.5 after) addition of the detergent and bleaching system.

The fabrics are laundered at 35°C (95°F) for a full cycle (12 min.) and rinsed at 21°C (70°F). The laundering method is repeated for 2,000 wash cycles without rupture of, or significant damage to, the natural rubber machine parts, or damage to the natural rubber articles.

### EXAMPLE IV (Not within the scope of the invention)

A granular detergent composition is prepared comprising the following ingredients.

| Component | Weight % |
|---|---|
| Anionic alkyl sulfate | 7 |
| Nonionic surfactant | 5 |
| Zeolite (0.1-10 micron) | 10 |
| Citrate | 2 |
| SKS-6 silicate builder | 10 |
| Acrylate maleate polymer | 4 |
| Nonanoyl caprolactam | 5 |
| Sodium percarbonate | 15 |
| Sodium carbonate | 5 |
| Ethylenediamine disuccinate chelant (EDDS) | 0.4 |
| Suds suppressor | 2 |
| Enzymes* | 1.5 |
| Soil release agent | 0.1 |
| Minors, filler** and water | Balance to 100% |

| | |
|---|---|
| *1:1:1 mixture of protease, lipase, and cellulase. | |
| **Can be selected from convenient materials such as CaCO₃, talc, clay, silicates, and the like. | |

In testing the bleaching performance and effect on natural rubber washing machine parts, the following test method is used:

Aqueous crutcher mixes of heat and alkali stable components of the detergent composition are prepared and spray-dried, and the other ingredients are admixed so that they contain the ingredients tabulated at the levels shown.

The detergent granules with bleach activator are added via the dispensing drawer together with 5 lb. (2.3 kg) of previously laundered fabrics to an automatic washing machine equipped with a natural rubber sump hose. Actual weights of detergent and bleach activator are taken to provide a 8,000 ppm concentration of the former and 400 ppm concentration of the latter in the 17 l water-fill machine. The water used has 85.6 mg/l (5 grains/gallon) hardness and a pH of 7 to 7.5 prior to (about 9 to about 10.5 after) addition of the detergent and bleaching system.

The fabrics are laundered at 40°C (104°F) for a full cycle (40 min.) and rinsed at 21°C (70°F). The laundering method is repeated for 2,000 wash cycles without rupture of, or significant damage to, the natural rubber parts.

### EXAMPLE VIII

A detergent composition is prepared by a procedure identical to that of Example IV, with the single exception that 6% of a 1:1 mixture of benzoyl caprolactam and 3,5,5-trimethylhexanoyl caprolactam is substituted for the nonanoyl caprolactam bleach activator. The laundering method of Example IV is repeated for 2,000 cycles without rupture of, or significant damage to, the natural rubber parts.

### EXAMPLE IX

A detergent composition is prepared by a procedure identical to that of Example IV, with the single exception that an equivalent amount of 3,5,5-trimethylhexanoyl caprolactam is substituted for the nonanoyl caprolactam. The laundering method of Example IV is repeated for 2,000 cycles without rupture of, or significant damage to, the natural rubber parts.

### EXAMPLE X

A detergent composition is prepared by a procedure identical to that of Example IV, with the exceptions that 15% of a 1:1 mixture of benzoyl caprolactam and nonanoyl caprolactam is substituted for the nonanoyl caprolactam bleach activator and the amount of sodium percarbonate is 25%. The laundering method of Example IV is repeated for 2,000 cycles without rupture of, or significant damage to, the natural rubber parts.

### EXAMPLE XI

A detergent composition is prepared by a procedure identical to that of Example IV, with the exception that 15% of a 1:1 mixture of nonanoyl caprolactam and tetraacetyl ethylene diamine (TAED) is substituted for the nonanoyl caprolactam bleach activator and the amount of sodium percarbonate is 25%. The laundering method of Example IV is repeated for 2,000 cycles without rupture of, or significant damage to, the natural rubber parts.

### EXAMPLE XII (Not within scope of the invention)

A bleaching system is prepared comprising the following ingredients.

| Component | Weight % |
|---|---|
| Nonanoyl caprolactam | 15 |
| Sodium percarbonate* | 25 |
| Ethylenediamine disuccinate chelant (EDDS) | 10 |
| Minors, filler** and water | Balance to 100% |

| | |
|---|---|
| * Average particle size of 400 to 1200 microns. | |
| **Can be selected from convenient materials such as CaCO₃, talc, clay, silicates, and the like. | |

The test method of Example IV is repeated with the single exception that the above bleaching system is substituted for the detergent composition of Example IV in an amount to provide a 500 ppm concentration in the 17 l water-fill machine. The laundering method of Example IV is repeated for 2,000 wash cycles without rupture of, or significant damage to, the natural rubber parts.

### EXAMPLE XIII (Not within the scope of the invention)

A bleaching system is prepared by a procedure identical to that of Example XII, with the single exception that an equivalent amount of a 1:1:1 mixture of octanoyl caprolactam, nonanoyl caprolactam, and decanoyl caprolactam is substituted for the nonanoyl caprolactam bleach activator. The laundering method of Example XII is repeated for 2,000 cycles without rupture of, or significant damage to, the natural rubber parts.

### EXAMPLE XV

A bleaching system is prepared by a procedure identical to that of Example XII, with the single exception that an equivalent amount of a 1:1 mixture of 3,5,5-trimethylhexanoyl caprolactam and a benzoxazin-type bleach activator, as disclosed in U.S. Pat. 4,966,723, is substituted for the nonanoyl caprolactam bleach activator. The laundering method of Example XII is repeated for 2,000 cycles without rupture of, or significant damage to, the natural rubber parts.

### EXAMPLE XVI (not within the scope of the invention)

A laundry bar suitable for hand-washing soiled fabrics is prepared by standard extrusion processes and comprises the following:

| Component | Weight % |
|---|---|
| C₁₂ linear alkyl benzene sulfonate | 30 |
| Phosphate (as sodium tripolyphosphate) | 7 |
| Sodium carbonate | 25 |
| Sodium pyrophosphate | 7 |
| Coconut monoethanolamide | 2 |
| Zeolite A (0.1-10 micron) | 5 |
| Carboxymethylcellulose | 0.2 |
| Polyacrylate (m.w. 1400) | 0.2 |
| Nonanoyl caprolactam | 5 |
| Sodium percarbonate | 5 |
| Brightener, perfume | 0.2 |
| Protease | 0.3 |
| CaSO₄ | 1 |
| MgSO₄ | 1 |
| Water | 4 |
| Filler* | Balance to 100% |

| | |
|---|---|
| *Can be selected from convenient materials such as CaCO₃, talc, clay, silicates, and the like. | |

The detergent laundry bars are processed in conventional soap or detergent bar making equipment as commonly used in the art.

The test method of Example III is repeated with the single exception that an equivalent amount of the above laundry bar composition is substituted for the detergent composition of Example III. The laundering method is repeated for 2,000 wash cycles without rupture of, or significant damage to, the natural rubber parts, or damage to the natural rubber articles.

### EXAMPLE XVIII

A laundry bar is prepared by a procedure identical to that of Example XVI, with the single exception that 6% of a 1:1 mixture of benzoyl caprolactam and a benzoxazin-type bleach activator, as disclosed in U.S. Pat. 4,966,723, is substituted for the nonanoyl caprolactam bleach activator. The laundering method of Example XVI is repeated for 2,000 cycles without rupture of, or significant damage to, the natural rubber parts, or damage to the natural rubber articles.

### EXAMPLE XIX

A laundry bar is prepared by a procedure identical to that of Example XVI, with the single exception that 6% of a 1:1 mixture of benzoyl caprolactam and tetraacetyl ethylene diamine is substituted for the nonanoyl caprolactam bleach activator. The laundering method of Example XVI is repeated for 2,000 cycles without rupture of, or significant damage to, the natural rubber parts, or damage to the natural rubber articles.

## Claims

1. A bleaching system which comprises a hydrophobic bleach activator, a hydrophilic bleach activator, and a peroxygen bleaching compound, preferably perborate or percarbonate; characterised in that said hydrophobic bleach activator is a bleach activator of the formula 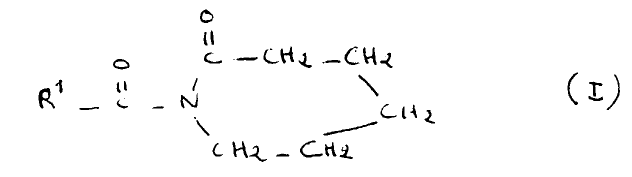 wherein R¹ is a hydrocarbyl substituent which contains at least 6 carbon atoms selected from the group consisting of benzoyl caprolactam, 3,5,5 trimethylhexanoyl caprolactam, nonanoyl caprolactam, decanoyl caprolactam and undecenoyl caprolactam.

2. A bleaching system according to Claim 1 wherein the hydrophilic bleach activator is a caprolactam activator of formula (I) wherein the R¹ substituent contains 6 or less carbon atoms, a tetraacetyl ethylene diamine, or a mixture thereof.

3. A bleaching system according to Claim 1 which additionally comprises a chelant, preferably ethylenediamine disuccinate chelant or an aminophosphonate chelant.

4. A bleaching system according to Claim 1 which comprises:
i) benzoyl caprolactam as a hydrophobic bleach activator;
ii) tetraacetyl ethylene diamine as a hydrophilic bleach activator;
iii) percarbonate as a peroxygen bleaching compound; and
iv) optionally, a bleach-stabilizing amount of a chelant.

5. A bleaching system according to Claim 1 which comprises:
i) nonanoyl caprolactam as a hydrophobic bleach activator;
ii) tetraacetyl ethylene diamine as a hydrophilic bleach activator;
iii) percarbonate as a peroxygen bleaching compound; and
iv) optionally, a bleach-stabilizing amount of a chelant.

6. A bleaching system according to Claim 1 which comprises:
i) 3,5,5-trimethylhexanoyl caprolactam as a hydrophobic bleach activator;
ii) tetraacetyl ethylene diamine as a hydrophilic bleach activator;
iii) percarbonate as a peroxygen bleaching compound; and
iv) optionally, a bleach-stabilizing amount of a chelant.

7. A laundry detergent composition comprising detersive surfactants, builders and detersive adjunct ingredients, characterized in that it comprises a bleaching system according to Claim 1.

8. A bleaching system which comprises benzoyl caprolactam bleach activator and a peroxygen bleaching compound, preferably selected from perborate salts and percarbonate salts.

9. A bleaching system according to Claim 8 which additionally comprises a chelant.

10. A caprolactam bleach activator compound of the formula: 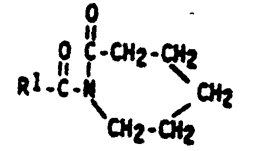 wherein 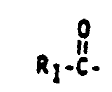 is selected from decanoyl, undecenoyl and 3,5,5-tri-methylhexanoyl.

11. A compound according to Claim 10 which is 3,5,5-tri-methylhexanoyl caprolactam.

12. A method for cleaning fabrics in automatic washing machines having parts made of natural rubber which is susceptible to oxidative degradation, said method comprising agitating said fabrics in said machine in an aqueous liquor comprising a detergent composition comprising conventional detergent ingredients and a bleaching system according to Claim 1 such that said natural rubber parts of said machine are substantially undamaged by the bleaching system.

## Patentansprüche

1. Bleichmittelsystem, umfassend einen hydrophoben Bleichaktivator, einen hydrophilen Bleichaktivator und eine Persauerstoff-Bleichmittelverbindung, vorzugsweise Perborat oder Percarbonat, dadurch gekennzeichnet, daß der hydrophobe Bleichaktivator ein Bleichaktivator der folgenden Formel ist: 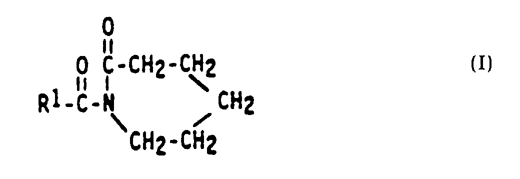 worin R¹ ein mindestens 6 Kohlenstoffatome enthaltender Kohlenwasserstoffsubstituent ist, gewählt aus der Benzoylcaprolactam, 3,5,5-Trimethylhexanoyl-caprolactam, Nonanoylcaprolactam, Decanoylcaprolactam und Undecenoylcaprolactam umfassenden Gruppe.

2. Bleichmittelsystem nach Anspruch 1, wobei der hydrophile Bleichaktivator ein Caprolactamaktivator der Formel (I), worin der R¹-Substituent 6 oder weniger Kohlenstoffatome enthält, ein Tetraacetylethylendiamin oder eine Mischung hiervon ist.

3. Bleichmittelsystem nach Anspruch 1, umfassend weiterhin einen Komplexbildner, vorzugsweise Ethylendiamindisuccinat-Komplexbildner oder ein Aminophosphonat-Komplexbildner.

4. Bleichmittelsystem nach Anspruch 1, umfassend
i) Benzoylcaprolactam als hydrophoben Bleichaktivator,
ii) Tetraacetylethylendiamin als hydrophilen Bleichaktivator,
iii) Percarbonat als Persauerstoff-Bleichmittelverbindung, und
iv) wahlweise eine bleichmittelstabilisierende Menge eines Komplexbildners.

5. Bleichmittelsystem nach Anspruch 1, umfassend
i) Nonanoylcaprolactam als hydrophoben Bleichaktivator,
ii) Tetraacetylethylendiamin als hydrophilen Bleichaktivator,
iii) Percarbonat als Persauerstoff-Bleichmittelverbindung, und
iv) wahlweise eine bleichmittelstabilisierende Menge eines Komplexbildners.

6. Bleichmittelsystem nach Anspruch 1, umfassend
i) 3,5,5-Trimethylhexanoylcaprolactam als hydrophoben Bleichaktivator,
ii) Tetraacetylethylendiamin als hydrophilen Bleichaktivator,
iii) Percarbonat als Persauerstoff-Bleichmittelverbindung, und
iv) wahlweise eine bleichmittelstabilisierende Menge eines Komplexbildners.

7. Wäschewaschmittelzusammensetzung, umfassend Waschtenside, Builder und Waschmittelzusatzbestandteile, dadurch gekennzeichnet, daß sie ein Bleichmittelsystem nach Anspruch 1 umfaßt.

8. Bleichmittelsystem, umfassend Benzoylcaprolactam-Bleichaktivator und eine Persauerstoff-Bleichmittelverbindung, vorzugsweise gewählt aus Perboratsalzen und Percarbonatsalzen.

9. Bleichmittelsystem nach Anspruch 8, umfassend zusätzlich einen Komplexbildner.

10. Caprolactam-Bleichaktivatorverbindung der Formel: 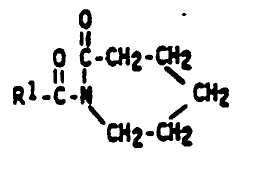 worin 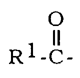 aus Decanoyl, Undecenoyl, und 3,5,5-Trimethylhexanoyl gewählt ist.

11. Verbindung nach Anspruch 10, nämlich 3,5,5-Trimethylhexanoylcaprolactam.

12. Verfahren zum Reinigen von Textilien in automatischen Waschmaschinen, welche Teile aufweisen, die aus natürlichem Kautschuk, der gegenüber oxidativem Abbau empfindlich ist, hergestellt sind, wobei das Verfahren das Bewegen der Textilien in der Maschine in einer wäßrigen Flotte, die eine Waschmittelzusammensetzung umfaßt, welche herkömmliche Waschmittelbestandteile und ein Bleichmittelsystem nach Anspruch 1 enthält, umfaßt, so daß die Teile aus natürlichem Kautschuk der Maschine durch das Bleichmittelsystem im wesentlichen unbeschädigt bleiben.

## Revendications

1. Système de blanchiment qui comprend un activateur de blanchiment hydrophobe, un activateur de blanchiment hydrophile et un composé de blanchiment peroxygéné, de préférence un perborate ou un percarbonate, caractérisé en ce que ledit activateur de blanchiment hydrophobe est un activateur de blanchiment de formule : 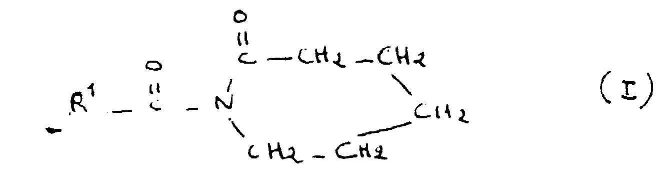 dans laquelle R¹ est un substituant hydrocarbyle qui contient au moins 6 atomes de carbone, choisi dans le groupe comprenant le benzoylcaprolactame, le 3,5,5-triméthylhexanoylcaprolactame, le nonanoylcaprolactame, le décanoylcaprolactame et l'undécénoylcaprolactame.

2. Système de blanchiment selon la revendication 1, dans lequel l'activateur de blanchiment hydrophile est un activateur de type caprolactame de formule (I), dans laquelle le substituant R¹ contient 6 atomes de carbone ou moins, une tétraacétyléthylènediamine, ou un de leurs mélanges.

3. Système de blanchiment selon la revendication 1, qui comprend en outre un agent chélatant, de préférence un agent chélatant de type disuccinate d'éthylènediamine ou un agent chélatant de type aminophosphonate.

4. Système de blanchiment selon la revendication 1, qui comprend :
i) du benzoylcaprolactame comme activateur de blanchiment hydrophobe.
ii) de la tétraacétyléthylènediamine comme activateur de blanchiment hydrophile,
iii) un percarbonate comme composé de blanchiment peroxygéné, et
iv) en option, une quantité d'un agent chélatant stabilisatrice d'agent de blanchiment.

5. Système de blanchiment selon la revendication 1, qui comprend :
i) du nonanoylcaprolactame comme activateur de blanchiment hydrophobe,
ii) de la tétraacétyléthylènediamine comme activateur de blanchiment hydrophile,
iii) un percarbonate comme composé de blanchiment peroxygéné, et
iv) en option, une quantité d'un agent chélatant stabilisatrice d'agent de blanchiment.

6. Système de blanchiment selon la revendication 1, qui comprend :
i) du 3,5,5-triméthylhexanoylcaprolactame comme activateur de blanchiment hydrophobe,
ii) de la tétraacétyléthylènediamine comme activateur de blanchiment hydrophile,
iii) un percarbonate comme composé de blanchiment peroxygéné, et
iv) en option, une quantité d'un agent chélatant stabilisatrice d'agent de blanchiment.

7. Composition détergente de lavage comprenant des agents tensioactifs détersifs, des adjuvants et des ingrédients d'addition détersifs, caractérisée en ce qu'elle comprend un système de blanchiment selon la revendication 1.

8. Système de blanchiment qui comprend un activateur de blanchiment à base de benzoylcaprolactame et un composé de blanchiment peroxygéné, de préférence choisi parmi les sels perborates et les sels percarbonates.

9. Système de blanchiment selon la revendication 8, qui comprend en outre un agent chélatant.

10. Composé activateur de blanchiment à base de caprolactame de formule : 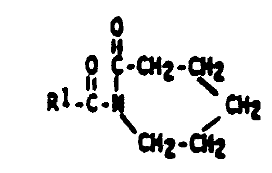 dans laquelle 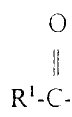 est choisi parmi les groupes décanoyle, undécénoyle et 3,5,5-triméthylhexanoyle.

11. Composé seion la revendication 10, qui est le 3,5,5-triméthylhexanoylcaprolactame.

12. Procédé de nettoyage de tissus dans des machines à laver automatiques ayant des parties constituées de caoutchouc naturel qui est susceptible de se dégrader par oxydation, ledit procédé comprenant l'agitation desdits tissus dans ladite machine dans une liqueur aqueuse comprenant une composition détergente contenant des ingrédients détergents classiques et un système de blanchiment selon la revendication 1. de façon que lesdites parties en caoutchouc naturel de ladite machine ne soient sensiblement pas endommagées par le système de blanchiment.
